(19) Europäisches Patentamt
European Patent Office
Office européen des brevets

(11) Veröffentlichungsnummer: 0 226 916
A2

(12) **EUROPÄISCHE PATENTANMELDUNG**

(21) Anmeldenummer: 86116980.3

(22) Anmeldetag: 06.12.86

(51) Int.Cl.[4]: C 07 D 249/08
C 07 D 233/60, A 01 N 43/653
A 01 N 43/56

(30) Priorität: 18.12.85 DE 3544731

(43) Veröffentlichungstag der Anmeldung: 01.07.87 Patentblatt 87/27

(84) Benannte Vertragsstaaten: AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Elbe, Hans-Ludwig, Dr.
Dasnöckel 59
D-5600 Wuppertal 11(DE)

(72) Erfinder: Büchel, Karl Heinz, Prof. Dr.
Dabringhausener Strasse 42
D-5093 Burscheid(DE)

(72) Erfinder: Reinecke, Paul, Dr.
Steinstrasse 8
D-5090 Leverkusen 3(DE)

(72) Erfinder: Brandes, Wilhelm, Dr.
Eichendorffstrasse 3
D-5653 Leichlingen 1(DE)

(72) Erfinder: Hänssler, Gerd, Dr.
Am Arenzberg 58a
D-5090 Leverkusen 3(DE)

(54) **1,4-Disubstituierte 1-Azolyl-3,3-dimethyl-butan-2-on- und -ol-Derivate.**

(57) Neue 1,4-disubstituierte 1-Azolyl-3,3-dimethyl-butan-2-on-und -ol-Derivate der Formel

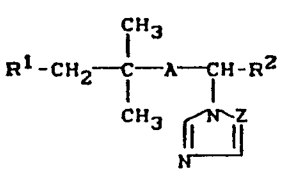

(I)

in welcher

$R^1$ für gegebenenfalls substituiertes Phenyl steht,

A für die Ketogruppe oder eine CH(OH)-Gruppierung steht,

Z für ein Stickstoffatom oder die CH-Gruppe steht und

$R^2$ für Halogenalkyl, Halogenalkenyl oder Halogenalkinyl steht,

sowie deren Säureadditionssalze und Metallsalzkomplexe, mehrere Verfahren zur Herstellung der neuen Stoffe und deren Verwendung als Fungizide.

EP 0 226 916 A2

0226916

BAYER AKTIENGESELLSCHAFT 5090 Leverkusen, Bayerwerk
Konzernverwaltung RP
Patentabteilung Dü/bo/c
Ib

1,4-Disubstituierte 1-Azolyl-3,3-dimethylbutan-2-on- und -ol-Derivate

Die vorliegende Erfindung betrifft neue 1,4-disubstituierte 1-Azolyl-3,3-dimethylbutan-2-on- und -ol-Derivate, mehrere Verfahren zu ihrer Herstellung sowie ihre Verwendung als Fungizide.

Es ist bereits bekannt geworden, daß bestimmte Azolylcarbonyl- und carbinol-Derivate fungizide Eigenschaften besitzen (vgl. DE-OS 30 48 266 und DE-OS 30 48 267). So lassen sich zum Beispiel 1-(2,6-Dichlorphenyl)-2,2-dimethyl-4-(1,2,4-triazol-1-yl)-hept-6-en-3-on, 2,2,5-Trimethyl-1-(4-chlorphenyl)-4-(1,2,4-triazol-1-yl)-hexan-3-ol, 2,2-Dimethyl-1-(2-methylphenyl)-4-(1,2,4-triazol-1-yl)-hept-6-en-3-on, 2,2-Dimethyl-1-phenyl-4-(1,2,4-triazol-1-yl)-octan-3-ol und 2,2-Dimethyl-1-phenyl-4-(1,2,4-triazol-1-yl)-hept-6-en-3-ol zur Bekämpfung von Pilzen einsetzen. Die Wirkung dieser Verbindungen ist jedoch in bestimmten Indikationsbereichen, insbesondere bei niedrigen Aufwandmengen und -konzentrationen, nicht immer ganz befriedigend.

Ferner ist bereits bekannt geworden, daß Zinkethylen-1,2-bisdithiocarbamidat gut zur Bekämpfung von pilzlichen Pflanzenkrankheiten eingesetzt werden kann (vergl. Phytopathology 33, S. 1113 (1963)). Die Verwendung dieses Stoffes ist aber dadurch beeinträchtigt, daß die Wirkung bei niedrigen Dosierungen nicht immer ausreichend ist.

Es wurden nun neue 1,4-disubstituierte 1-Azolyl-3,3-dimethylbutan-2-on- und -ol-Derivate der Formel

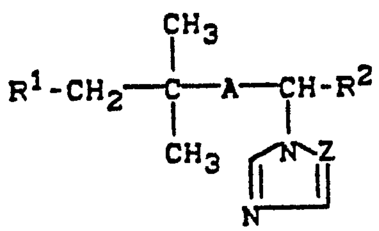

(I)

in welcher

$R^1$ für gegebenenfalls substituiertes Phenyl steht,

A für die Ketogruppe oder eine CH(OH)-Gruppierung steht,

Z für ein Stickstoffatom oder die CH-Gruppe steht und

$R^2$ für Halogenalkyl, Halogenalkenyl oder Halogenalkinyl steht,

sowie deren Säureadditionssalze und Metallsalzkomplexe gefunden.

Die Verbindungen der Formel (I), in denen A für die CH(OH)-Gruppe steht, besitzen zwei benachbarte asymmetrisch substituierte Kohlenstoffatome. Sie können deshalb in den beiden geometrischen Isomeren (threo- und erythro-Form) vorliegen, die in unterschiedlichen Mengenverhältnissen anfallen können. Die Verbindungen der Formel (I), in denen A für die Ketogruppe steht, besitzen ein asymmetrisch substituiertes Kohlenstoffatom. In beiden Fällen können die Verbindungen der Formel (I) in verschiedenen optischen Isomerenformen vorliegen, die in unterschiedlichen Mengenverhältnissen anfallen können. Sämtliche Isomeren werden erfindungsgemäß beansprucht.

Weiterhin wurde gefunden, daß man die neuen 1,4-disubstituierten 1-Azolyl-3,3-dimethylbutan-2-on- und -ol-Derivate der Formel (I) sowie deren Säureadditionssalze und Metallsalzkomplexe erhält, wenn man

(a) Azolyl-ketone der Formel

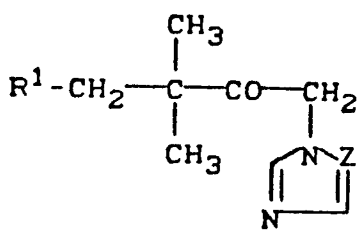

(II)

in welcher

$R^1$ und Z die oben angegebenen Bedeutungen haben,

mit einer Verbindung der Formel

$$R^2—Y \qquad (III)$$

in welcher

$R^2$ die oben angegebene Bedeutung hat und

Y für eine elektronenanziehende Abgangsgruppe steht,

in Gegenwart einer Base und in Gegenwart eines Verdünnungsmittels oder in einem wässrig-organischen Zweiphasensystem in Gegenwart eines Phasentransferkatalysators umsetzt;

oder

(b) Acetylen-Verbindungen der Formel

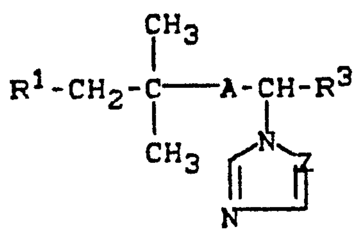

(IV)

in welcher

$R^1$, A und Z die oben angegebenen Bedeutungen haben und

$R^3$ für Alkinyl steht,

mit Halogen in Gegenwart eines Verdünnungsmittels und in Gegenwart einer Base umsetzt, und gegebenenfalls

(c) die nach den Verfahren (a) oder (b) erhaltenen Ketone der Formel

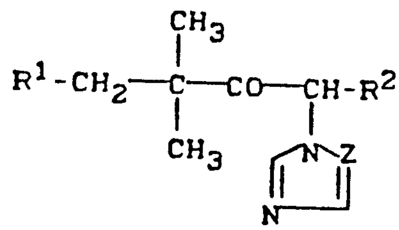

(Ia)

in welcher

$R^1$, Z und $R^2$ die oben angegebenen Bedeutungen haben,

in üblicher Weise reduziert

und gegebenenfalls an die so erhaltenen Verbindungen der Formel (I) anschließend eine Säure oder ein Metallsalz addiert.

Schließlich wurde gefunden, daß die neuen 1,4-disubstituierten 1-Azolyl-3,3-dimethylbutan-2-on- und -ol-Derivate der Formel (I) sowie deren Säureadditions-Salze und Metallsalz-Komplexe sehr gute fungizide Eigenschaften besitzen.

Überraschenderweise zeichnen sich die erfindungsgemäßen Stoffe durch eine bessere fungizide Wirkung aus als die

konsitutionell ähnlichen, aus dem Stand der Technik bekannten Verbindungen gleicher Indikation. So übertreffen die erfindungsgemäßen Stoffe zum Beispiel 1-(2,6-Dichlorphenyl)-2,2-dimethyl-4-(1,2,4-triazol-1-yl)-hept-6-en-3-on, 2,2,5-Trimethyl-1-(4-chlorphenyl)-4-(1,2,4-triazol-1-yl)-hexan-3-ol, 2,2-Dimethyl-1-(2-methylphenyl)-4-(1,2,4-triazol-1-yl)-hept-6-en-3-on, 2,2-Dimethyl-1-phenyl-4-(1,2,4-triazol-1-yl)-octan-3-ol und 2,2-Dimethyl-1-phenyl-4-(1,2,4-triazol-1-yl)-hept-6-en-3-ol bezüglich ihrer fungiziden Eigenschaften. Darüber hinaus besitzen die erfindungsgemäßen Stoffe auch eine deutlich bessere fungizide Wirksamkeit als das Zinkethylen-1,2-bis-dithiocarbamidat, welches ein bekannter Wirkstoff gleicher Wirkungsrichtung ist.

Außerdem sind die neuen 1,4-disubstituierten 1-Azolyl-3,3-dimethyl-butan-2-one der Formel (Ia) interessante Zwischenprodukte zur Herstellung von weiteren Pflanzenschutz-Wirkstoffen. So können durch entsprechende Umsetzungen funktionelle Derivate der Ketogruppe erhalten werden wie z. B. Oxime und Oximether, Hydrazone und Ketale.

Im übrigen sind auch die neuen 1,4-disubstituierten 1-Azolyl-3,3-dimethyl-butan-2-ole interessante Zwischenprodukte zur Herstellung von weiteren Pflanzenschutz-Wirkstoffen. So können z. B. diese Verbindungen durch Umsetzung an der Hydroxy-Gruppe in üblicher Weise in die entsprechenden Ether überführt werden. Weiterhin können durch Umsetzung mit z. B. Acylhalogeniden oder Carbamoylchloriden in prinzipiell bekannter Weise Acyl- oder Carbamoyl-Derivate der Alkohole der Formel (I) erhalten werden.

Die erfindungsgemäßen 1,4-disubstituierten 1-Azolyl-3,3-dimethylbutan-2-on- und -ol-Derivate sind durch die Formel (I) allgemein definiert. Bevorzugt sind Verbindungen der Formel (I), in denen

$R^1$ für gegebenenfalls einfach bis vierfach, gleich oder verschieden substituiertes Phenyl steht, wobei als Substituenten genannt seien: Halogen, Alkyl, Alkoxy und Alkylthio mit jeweils 1 bis 6 Kohlenstoffatomen, Halogenalkyl, Halogenalkoxy und Halogenalkylthio mit jeweils 1 bis 4 Kohlenstoffatomen und 1 bis 5 gleichen oder verschiedenen Halogenatomen, Nitro, Cyano, Alkoxycarbonyl mit 1 bis 4 Kohlenstoffatomen im Alkoxyteil sowie gegebenenfalls durch Halogen substituiertes Phenyl und Phenoxy,

A für die Ketogruppe oder eine CH(OH)-Gruppierung steht,

Z für ein Stickstoffatom oder die CH-Gruppe steht und

$R^2$ für geradkettiges oder verzweigtes Halogenalkyl mit 2 bis 12 Kohlenstoffatomen und Halogenalkenyl mit 3 bis 12 Kohlenstoffatomen mit jeweils 1 bis 3 gleichen oder verschiedenen Halogenatomen oder für geradkettiges oder verzweigtes Halogenalkinyl mit 3 bis 12 Kohlenstoffatomen und einem Halogenatom steht, wobei die Dreifachbindung und das Halogenatom endständig sind.

Besonders bevorzugt sind diejenigen Verbindungen der Formel (I), in denen

$R^1$ für gegebenenfalls einfach bis dreifach, gleich oder verschieden substituiertes Phenyl steht, wobei als Substituenten genannt seien: Fluor, Chlor, Brom, Iod, Methyl, Ethyl, Isopropyl, tert.-Butyl, Methoxy, Methylthio, Trifluormethyl, Trifluormethoxy, Chlordifluormethoxy, Trifluormethylthio, Chlordifluormethylthio, Nitro, Cyano, Methoxycarbonyl, gegebenenfalls durch Fluor und/oder Chlor substituiertes Phenyl und gegebenenfalls durch Fluor und/oder Chlor substituiertes Phenoxy,

A für die Ketogruppe oder eine CH(OH)-Gruppierung steht,

Z für ein Stickstoffatom oder die CH-Gruppe steht und

$R^2$ für geradkettiges oder verzweigtes Halogenalkyl mit 2 bis 8 Kohlenstoffatomen und 1 bis 3 gleichen oder verschiedenen Halogenatomen wie Fluor, Chlor, Brom und Iod steht, ferner für geradkettiges oder verzweigtes Halogenalkenyl mit 3 bis 8 Kohlenstoffatomen und 1 bis 3 gleichen oder verschiedenen Halogenatomen wie Fluor, Chlor, Brom und Iod steht, oder für geradkettiges oder verzweigtes Halogenalkinyl mit 3 bis 8 Kohlenstoffatomen und 1 Halogenatom, wie Fluor, Chlor, Brom und Iod, steht, wobei die Dreifachbindung und das Halogenatom endständig sind.

Bevorzugte erfindungsgemäße Verbindungen sind auch Additionsprodukte aus Säuren und denjenigen 1,4-disubstituierten 1-Azolyl-3,3-dimethyl-butan-2-on- und -ol-Derivaten der Formel (I), in denen die Substituenten $R^1$, A, Z und $R^2$ die Bedeutungen haben, die bereits vorzugsweise für diese Substituenten genannt wurden.

Zu den Säuren, die addiert werden können, gehören vorzugsweise Halogenwasserstoffsäuren wie z. B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren wie z. B. Essigsäure, Maleinsäure, Bernsteinsäure, Fumarsäure, Weinsäure, Zitronensäure, Salizylsäure, Sorbinsäure und Milchsäure sowie Sulfonsäuren wie z. B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen Verbindungen der Formel (I), in denen die Substituenten $R^1$, A, Z und $R^2$ die Bedeutungen haben, die bereits vorzugsweise für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die

Halogenwasserstoffsäuren wie z. B. die Chlorwasserstoffsäure und die Bromwasserstoffsäure, ferner Phosphorsäure, Salpetersäure und Schwefelsäure.

Im einzelnen seien außer den bei den Herstellungsbeispielen genannten Verbindungen die folgenden Verbindungen der Formel

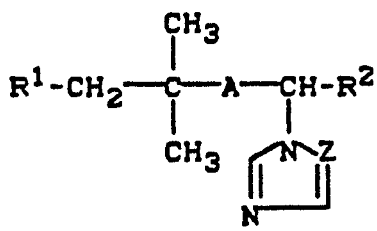

(I)

in denen

A für die Ketogruppe oder eine CH(OH)-Gruppierung steht und

Z für ein Stickstoffatom oder die CH-Gruppe steht,

genannt:

Tabelle 1

| $R^1$ | $R^2$ |
| --- | --- |
| 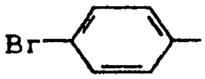 | $-CH_2-\underset{\displaystyle Cl}{\underset{\vert}{C}}=CH_2$ |
| 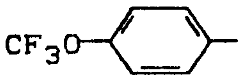 | $-CH_2-\underset{\displaystyle Cl}{\underset{\vert}{C}}=CH_2$ |
| 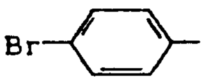 | $-CH_2-CH_2-CH_2-CH_2-Cl$ |
| 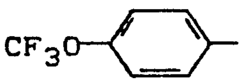 | $-CH_2-CH_2-CH_2-CH_2-Cl$ |
| 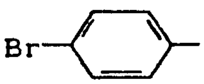 | $-CH_2-\underset{\displaystyle Cl}{\underset{\vert}{C}}=CH-CH_3$ |
| 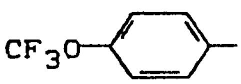 | $-CH_2-\underset{\displaystyle Cl}{\underset{\vert}{C}}=CH-CH_3$ |
| 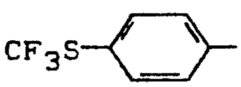 | $-CH_2-\underset{\displaystyle Cl}{\underset{\vert}{C}}=CH_2$ |
| 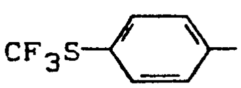 | $-CH_2-\underset{\displaystyle Cl}{\underset{\vert}{C}}=CH-CH_3$ |
| 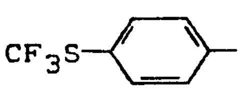 | $-CH_2-CH_2-CH_2-CH_2-Cl$ |
| 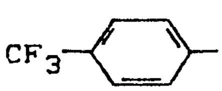 | $-CH_2-CH=C\genfrac{}{}{0pt}{}{\diagup CH_3}{\diagdown Cl}$ |

0226916

<u>Tabelle 1</u> (Fortsetzung)

| $R^1$ | $R^2$ |
|---|---|
| 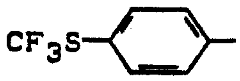 | $-CH_2-C\equiv C-I$ |
| 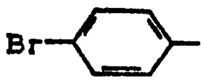 | $-CH_2-C\equiv C-I$ |
| 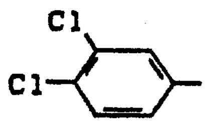 | $-CH_2-C\equiv C-I$ |
| 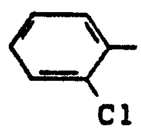 | $-CH_2-C\equiv C-I$ |
| 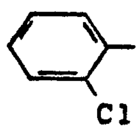 | $-CH_2-CH=C\langle{}^{Cl}_{CH_3}$ |
| 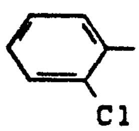 | $-CH_2-\underset{\underset{Cl}{\mid}}{C}H=CH-CH_3$ |
| 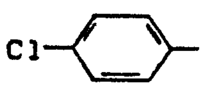 | $-CH_2-\underset{\underset{Cl}{\mid}}{C}=CH_2$ |
| 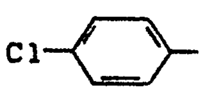 | $-CH_2-\underset{\underset{Cl}{\mid}}{C}=CH-CH_3$ |
| 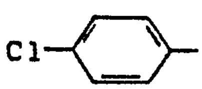 | $-CH_2-CH_2-CH_2-I$ |

Tabelle 1 (Fortsetzung)

| $R^1$ | $R^2$ |
|---|---|
| 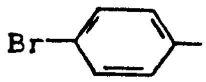 | $-CH_2-CH_2-CH_2-I$ |
| 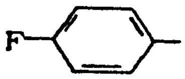 | 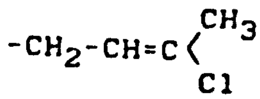 |
| 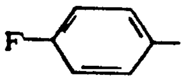 | 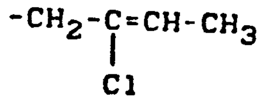 |
| 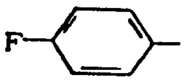 | 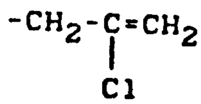 |
| 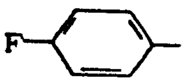 | $-CH_2-C\equiv C-I$ |
| 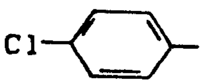 | $-CH_2-C\equiv C-I$ |
| 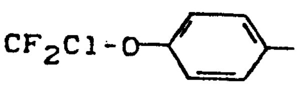 | 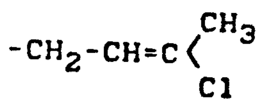 |
| 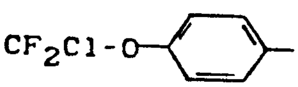 | $-CH_2-C\equiv C-I$ |
| 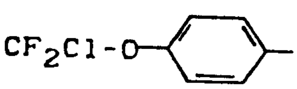 | $-CH_2-CH=CHCl$ |
| 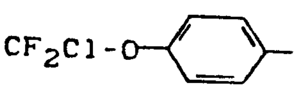 | 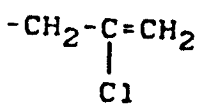 |

Tabelle 1 (Fortsetzung)

| $R^1$ | $R^2$ |
|---|---|
| 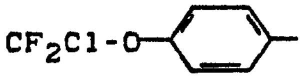 | 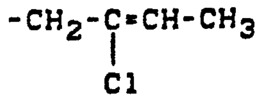 |
| 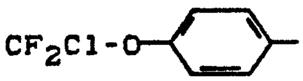 | $-CH_2-CH_2-CH_2-CH_2-Cl$ |
| 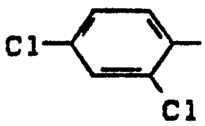 | 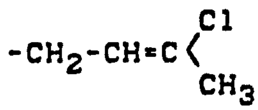 |
| 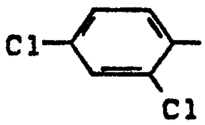 | 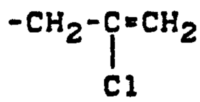 |
| 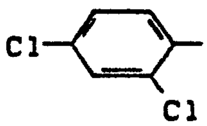 | 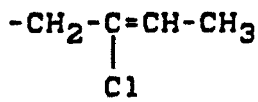 |
| 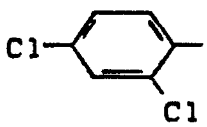 | $-CH_2-CH=CH-Cl$ |
| 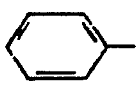 | 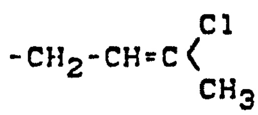 |
| 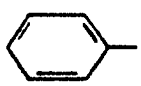 | 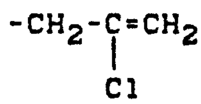 |
| 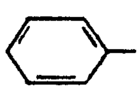 | 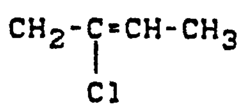 |

Tabelle 1 (Fortsetzung)

| $R^1$ | $R^2$ |
|---|---|
| 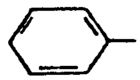 | $-CH_2-C \equiv C-I$ |
| 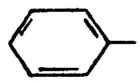 | $-CH_2-CH_2-CH_2-I$ |
| 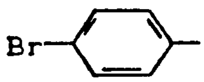 | $-CH_2-C \equiv C-I$ |
| 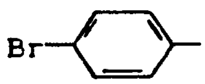 | $-CH_2-C \equiv C-Br$ |
| 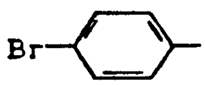 | $-CH_2-C \equiv C-Cl$ |
| 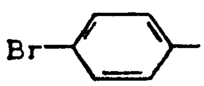 | $-CH_2-CH_2-C \equiv C-Cl$ |
| 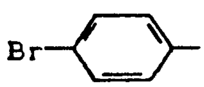 | $-CH_2-CH_2-C \equiv C-Br$ |
| 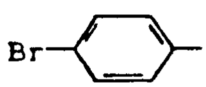 | $-CH_2-CH_2-C \equiv C-I$ |
| 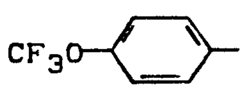 | $-CH_2-C \equiv C-Br$ |
| 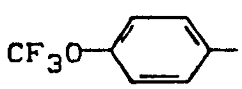 | $-CH_2-C \equiv C-I$ |
| 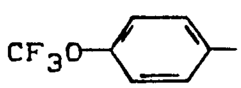 | $-CH_2-C \equiv C-Cl$ |

Tabelle 1 (Fortsetzung)

| $R^1$ | $R^2$ |
|---|---|
| 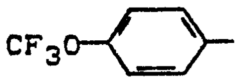 | $-CH_2-CH_2-C{\equiv}C-Cl$ |
| 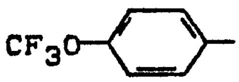 | $-CH_2-CH_2-C{\equiv}C-Br$ |
| 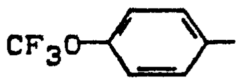 | $-CH_2-CH_2-C{\equiv}C-I$ |
| 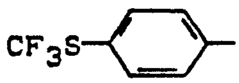 | $-CH_2-C{\equiv}C-Br$ |
| 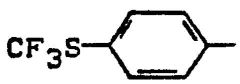 | $-CH_2-C{\equiv}C-Cl$ |
| 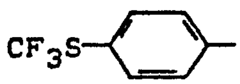 | $-CH_2-CH_2-C{\equiv}C-Cl$ |
| 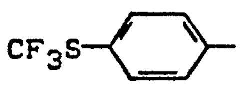 | $-CH_2-CH_2-C{\equiv}C-Br$ |
| 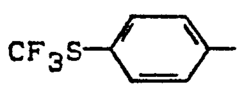 | $-CH_2-CH_2-C{\equiv}C-I$ |
| 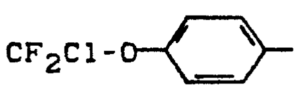 | $-CH_2-C{\equiv}C-Br$ |
| 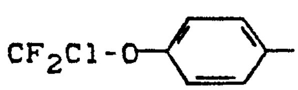 | $-CH_2-C{\equiv}C-Cl$ |

Tabelle 1 (Fortsetzung)

| $R^1$ | $R^2$ |
|---|---|
| 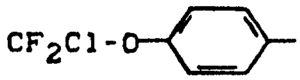 | $-CH_2-CH_2-C{\equiv}C-Cl$ |
| 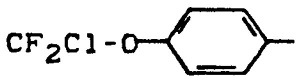 | $-CH_2-CH_2-C{\equiv}C-Br$ |
| 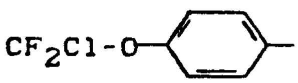 | $-CH_2-CH_2-C{\equiv}C-I$ |

Verwendet man beispielsweise 4-(4-Bromphenyl)-3,3-dimethyl-1-(1,2,4-triazol-1-yl)-butan-2-on und 1,3-Dichlorbut-2-en als Ausgangsstoffe, so kann der Ablauf des erfindungsgemäßen Verfahrens (a) durch das folgende Formelschema wiedergegeben werden:

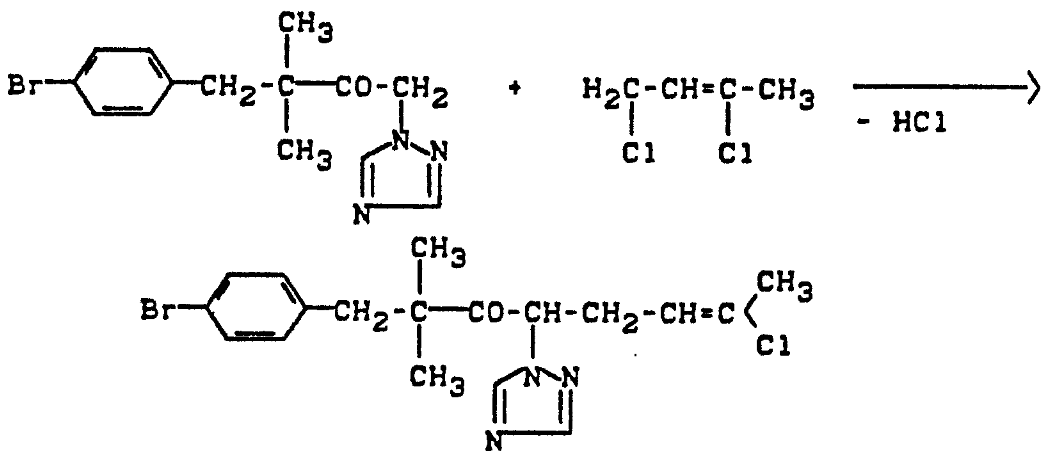


Verwendet man beispielsweise 1-(2,4-Dichlorphenyl)-2,2-dimethyl-4-(1,2,4-triazol-1-yl)-hept-6-in-3-ol und Iod als Ausgangsstoffe und wäßrige Natronlauge als Base, so kann der Verlauf des erfindungsgemäßen Verfahrens (b) durch das folgende Formelschema wiedergegeben werden:

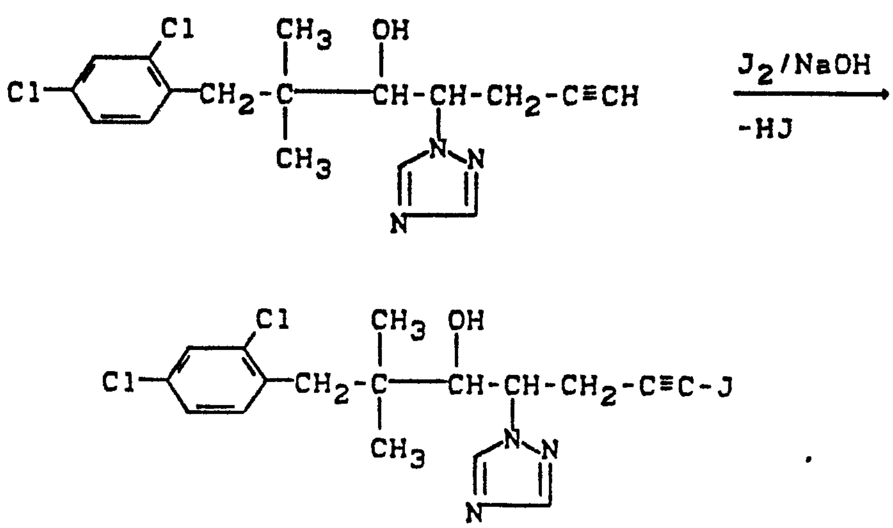

Verwendet man beispielsweise 1-(4-Bromphenyl)-7-chlor-2,2-dimethyl-4-(1,2,4-triazol-1-yl)-oct-6-en-3-on als Ausgangsstoff und Natriumborhydrid als Reduktionsmittel, so kann der Ablauf des erfindungsgemäßen Verfahrens (c) durch das folgende Formelschema wiedergegeben werden:

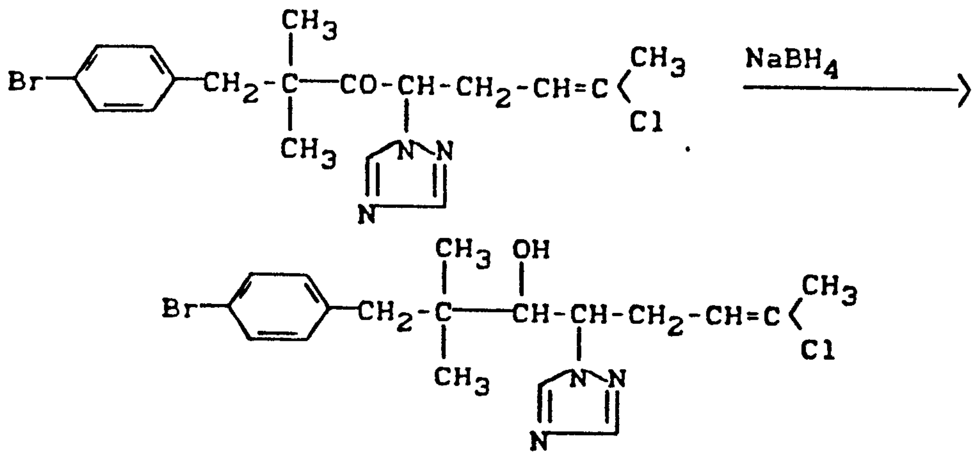


Die für die Durchführung des erfindungsgemäßen Verfahrens (a) als Ausgangsstoffe benötigten Azolyl-ketone sind durch die Formel (II) allgemein definiert. In dieser Formel haben $R^1$ und Z vorzugsweise die Bedeutungen, die bereits im Zusammenhang mit der Beschreibung der erfindungsgemäßen Stoffe der Formel (I) vorzugsweise für diese Substituenten genannt wurden.

Die Azolyl-ketone der Formel (II) sind teilweise bekannt (vergl. DE-OS 30 48 266). Sie können erhalten werden, indem man Halogenketone der Formel

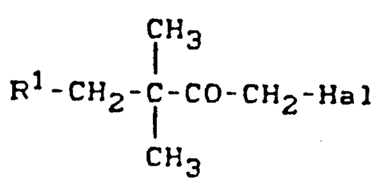

(V)

in welcher

$R^1$ die oben angegebene Bedeutung hat und

Hal für Halogen, insbesondere für Chlor oder Brom steht,

mit Azolen der Formel

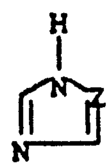

(VI)

in welcher

Z die oben angegebene Bedeutung hat,

in Gegenwart eines Verdünnungsmittels und in Gegenwart eines Säurebindemittels umsetzt.

Als Verdünnungsmittel kommen bei der obigen Herstellung von Azolyl-ketonen der Formel (II) inerte organische Lösungsmittel in Frage. Hierzu gehören vorzugsweise Ketone wie Diethylketon und insbesondere Aceton und Methylethylketon; Nitrile, wie Propionitril und Acetonitril; Alkohole, wie Ethanol oder Isopropanol; Ether, wie Tetrahydrofuran oder Dioxan; aromatische Kohlenwasserstoffe, wie Toluol, Benzol oder Chlorbenzol; Formamide, wie Dimethylformamid; und halogenierte Kohlenwasserstoffe.

Als Säurebindemittel kann man alle üblicherweise verwendbaren anorganischen oder organischen Säurebinder zugeben, wie Alkalimetallcarbonate, beispielsweise Natriumcarbonat,

Kaliumcarbonat und Natriumhydrogencarbonat, oder niedere tertiäre Alkylamine, Cycloalkylamine oder Aralkylamine, beispielsweise Triethylamin, N,N-Dimethylcyclohexylamin, Dicyclohexylamin, N,N-Dimethylbenzylamin, weiterhin Pyridin und Diazabicyclooctan. Vorzugsweise verwendbar ist auch ein Überschuß an Azol.

Die Reaktionstemperaturen können bei der obigen Umsetzung in einem größeren Bereich variiert werden. Im allgemeinen arbeitet man zwischen 20 bis 150 °C, vorzugsweise zwischen 40 und 120 °C.

Bei der Herstellung der Azolylketone der Formel (II) setzt man auf 1 Mol Halogenketon der Formel (V) vorzugsweise 2 bis 4 Mol Azol der Formel (VI) und 1 bis 4 Mol Säurebinder ein. Zur Isolierung der Verbindungen der Formel (II) wird das Lösungsmittel abdestilliert und der Rückstand in üblicher Weise aufgearbeitet.

Die Halogenketone der Formel (V) sind teilweise bekannt (vergl. DE-OS 30 48 266). Sie lassen sich herstellen, indem man Ketone der Formel

$$\begin{array}{c} \quad\quad\quad\quad\quad CH_3 \\ \quad\quad\quad\quad\quad | \\ R^1-CH_2-C-CO-CH_3 \\ \quad\quad\quad\quad\quad | \\ \quad\quad\quad\quad\quad CH_3 \end{array} \qquad (VII)$$

in welcher

$R^1$ die oben angegebene Bedeutung hat,

mit Chlor oder Brom in Gegenwart eines inerten organischen Lösungsmittels, wie beispielsweise Ether, chlorierten oder nicht chlorierten Kohlenwasserstoffen, bei Raumtemperatur umsetzt, oder mit üblichen Chlorierungsmitteln, wie beispielsweise Sulfurylchlorid, bei Temperaturen zwischen 20 und 60 °C umsetzt.

Die Ketone der Formel (VII) sind teilweise bekannt (vergl. DE-OS 30 48 266 und DE-OS 32 10 725). Sie können erhalten werden, indem man Methylisopropylketon mit Halogeniden der Formel

$$R^1\text{-}CH_2\text{-}Hal \quad (VIII)$$

in welcher

$R^1$ die oben angegebene Bedeutung hat und

Hal für Halogen, wie Chlor oder Brom, steht,

in Gegenwart einer Base, wie beispielsweise einem Alkali- oder Erdalkalimetallhydroxid, wie pulverisiertes Kalium- und Natriumhydroxid, und in Gegenwart eines Verdünnungsmittels, wie beispielsweise eines (cyclo)aliphatischen oder gegebenenfalls chlorierten aromatischen Kohlenwasserstoffes, wie Benzol, Xylol, Chlorbenzol, Dichlorbenzol, Cyclohexan, Petrolether, Pentan, Hexan oder Toluol, sowie in Gegenwart eines Phasentransferkatalysators, wie beispielsweise eines Derivates von einem Ammoniumsalz, wobei Triethylbenzylammonium-chlorid, Tetrabutylammonium-iodid, -bromid oder -chlorid speziell genannt seien, bei Temperaturen zwischen 0 °C und 150 °C umsetzt.

Methylisopropylketon und die Halogenide der Formel (VIII) sind allgemein bekannte Verbindungen der organischen Chemie.

Die Azole der Formel (VI) sind ebenfalls allgemein bekannte Verbindungen der organischen Chemie.

Die außerdem für das erfindungsgemäße Verfahren (a) als Ausgangsstoffe zu verwendenden Verbindungen sind durch die Formel (III) allgemein definiert. In dieser Formel steht $R^2$ vorzugsweise für diejenigen Reste, die bereits im Zusammenhang mit der Beschreibung der erfindungsgemäßen Stoffe der Formel (I) vorzugsweise für diesen Substituenten genannt wurden. Y steht vorzugsweise für Chlor, Brom, Iod, p-Methylphenylsulfonyloxy, die Gruppierung $-O-SO_2-OR$ oder $NR_3$, in welcher R für Methyl, Ethyl, n- oder i-Propyl steht.

Die Verbindungen der Formel (III) sind allgemein bekannte Stoffe der organischen Chemie.

Für das erfindungsgemäße Verfahren (a) kommen als Verdünnungsmittel inerte organische Lösungsmittel in Frage. Hierzu gehören vorzugsweise aromatische Kohlenwasserstoffe, wie Benzol, Toluol oder Xylol; halogenierte Kohlenwasserstoffe, wie Methylenchlorid, Tetrachlorkohlenstoff, Chloroform oder Chlorbenzol; Ester, wie Essigester; Formamide, wie Dimethylformamid, sowie Dimethylsulfoxid.

Das erfindungsgemäße Verfahren (a) wird in Gegenwart einer Base durchgeführt. Hierbei können alle üblichen organischen und insbesondere anorganischen Basen eingesetzt werden. Vorzugsweise verwendbar sind Alkalimetallhydroxide oder Alkalimetallcarbonate, wie Natrium- und Kaliumhydroxid, Natriumcarbonat und Kaliumcarbonat.

Die Reaktionstemperaturen können bei der Durchführung des erfindungsgemäßen Verfahrens (a) in einem größeren Bereich variiert werden. Im allgemeinen arbeitet man bei Temperaturen zwischen 0° C und 100 °C, vorzugsweise zwischen 20 und 100 °C.

Bei der Durchführung des erfindungsgemäßen Verfahrens (a) setzt man vorzugsweise auf 1 Mol der Verbindung der Formel (II) 1 bis 1,2 Mol einer Verbindung der Formel (III) ein. Die Isolierung der Endprodukte der Formel (I) erfolgt nach üblichen Methoden.

Das erfindungsgemäße Verfahren (a) kann auch in einem Zweiphasensystem, wie beispielsweise einem System aus wäßriger Natron- oder Kalilauge/Toluol oder Methylenchlorid, gegebenenfalls unter Zusatz von 0,1 bis 1 Mol eines Phasen-Transfer-Katalysators, beispielsweise einer Ammonium- oder Phosphoniumverbindung, wie Benzyldodecyldimethyl-ammoniumchlorid oder Triethyl-benzyl-ammoniumchlorid, durchgeführt werden.

Die bei dem Verfahren (b) als Ausgangsstoffe benötigten Acetylen-Verbindungen sind durch die Formel (IV) allgemein definiert. In dieser Formel haben $R^1$, A und Z vorzugsweise diejenigen Bedeutungen, die bereits im Zusammenhang mit der Beschreibung der erfindungsgemäßen Stoffe der Formel

(I) vorzugsweise für diese Reste genannt wurden. $R^3$ steht vorzugsweise für geradkettiges oder verzweigtes Alkinyl mit 3 bis 12, insbesondere 3 bis 8 Kohlenstoffatomen, wobei die Dreifachbindung endständig ist.

Die Acetylen-Verbindungen der Formel (IV) sind teilweise bekannt (vgl. DE-OS 30 48 266). Man erhält Acetylen-Verbindungen der Formel (IV), indem man Azolyl-ketone der Formel (II) mit Alkinyl-Verbindungen der Formel

$$R^3-Y \qquad (IX)$$

in welcher

$R^3$ und Y die oben angegebenen Bedeutungen haben,

unter den Bedingungen umsetzt, die auch im Falle des erfindungsgemäßen Verfahrens (a) angewandt werden.

Als Reaktionskomponenten dienen bei dem erfindungsgemäßen Verfahren (b) Halogene. Vorzugsweise verwendbar sind Chlor, Brom und Iod.

Als Verdünnungsmittel können bei der Durchführung des erfindungsgemäßen Verfahrens (b) alle üblichen, für derartige Reaktionen geeigneten Solventien eingesetzt werden. Vorzugsweise verwendbar sind Alkohole, wie Methanol, Ethanol, n-Propanol, iso-Propanol, n-Butanol, iso-Butanol und tert.-Butanol.

Als Basen kommen bei der Durchführung des erfindungsgemäßen Verfahrens (b) vorzugsweise Lösungen von starken anorganischen Basen in Betracht. Besonders bevorzugt sind wäßrige Lösungen von Alkalimetallhydroxiden, wie Natriumhydroxid und Kaliumhydroxid.

Die Reaktionstemperaturen können auch bei der Durchführung des erfindungsgemäßen Verfahrens (b) innerhalb eines größeren Bereiches variiert werden. Im allgemeinen arbeitet man bei Temperaturen zwischen 0°C und 40°C, vorzugsweise bei Temperaturen zwischen 0°C und 25°C.

Bei der Durchführung des erfindungsgemäßen Verfahrens (b) setzt man auf 1 Mol an Acetylen-Verbindung der Formel (IV) im allgemeinen 1 bis 10 Mol Halogen und 1 bis 10 Mol an Base, vorzugsweise 1 bis 3 Mol Halogen und 1 bis 3 Mol Base, ein. Die Reaktionszeit beträgt im allgemeinen zwischen 2 und 48 Stunden, vorzugsweise zwischen 8 und 24 Stunden. Die Aufarbeitung erfolgt nach üblichen Methoden.

Die bei der Durchführung des erfindungsgemäßen Verfahrens (c) als Ausgangsstoffe benötigten Verbindungen sind durch die Formel (Ia) definiert. Diese Stoffe lassen sich nach dem erfindungsgemäßen Verfahren (a) herstellen.

Die erfindungsgemäße Reduktion gemäß Verfahren (c) erfolgt nach üblichen Methoden, z.B. durch Umsetzung von Verbindungen der Formel (Ia) mit komplexen Hydriden, gegebenenfalls in Gegenwart eines Verdünnungsmittels, oder mit Aluminiumisopropylat in Gegenwart eines Verdünnungsmittels.

Als komplexe Hydride kommen vorzugsweise Natriumborhydrid und Lithiumaluminiumhydrid in Frage.

Arbeitet man mit komplexen Hydriden, so kommen als Verdünnungsmittel für die erfindungsgemäße Umsetzung nach Verfahren (c) polare organische Lösungsmittel in Frage. Hierzu gehören vorzugsweise Alkohole, wie Methanol, Ethanol, Butanol, Isopropanol und Ether, wie Diethylether oder Tetrahydrofuran.

Die Reaktion wird im allgemeinen bei Temperaturen zwischen 0 und 30 °C, vorzugsweise zwischen 0 und 20 °C, durchgeführt. Hierzu setzt man auf 1 Mol eines Ketons der Formel (Ia) etwa 1 Mol eines komplexen Hydrids, wie Natiumborhydrid oder Lithiumalanat, ein. Zur Isolierung der reduzierten Verbindungen der Formel (I) wird der Rückstand in verdünnter Säure, z.B. Salzsäure, aufgenommen, anschließend alkalisch gestellt und mit einem organischen Lösungsmittel extrahiert. Die weitere Aufarbeitung erfolgt in üblicher Weise.

Arbeitet man bei dem erfindungsgemäßen Verfahren (c) mit Aluminiumisopropylat, so kommen als Verdünnungsmittel für die Umsetzung bevorzugt Alkohole, wie Isopropanol, oder inerte Kohlenwasserstoffe, wie Benzol, in Frage. Die Reaktionstemperaturen können wiederum in einem größeren Bereich variiert werden. Im allgemeinen arbeitet man bei Temperaturen zwischen 20 und 120 °C, vorzugsweise zwischen 50 und 100 °C. Zur Durchführung der Reaktion setzt man auf 1 Mol eines Ketons der Formel (Ia) etwa 1 bis 2 Mol Aluminiumisopropylat ein. Zur Isolierung der reduzierten Verbindungen der Formel (I) wird das überschüssige Lösungsmittel durch Destillation im Vakuum entfernt, und das entstandene Produkt mit verdünnter Säure, wie Schwefelsäure, oder Base, wie wäßriger Natronlauge, behandelt. Die weitere Aufarbeitung erfolgt in üblicher Weise.

Bei der Durchführung der erfindungsgemäßen Verfahren (a) bis (c) arbeitet man im allgemeinen unter Normaldruck. Es ist jedoch auch möglich, unter erhöhtem oder vermindertem Druck zu arbeiten.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z. B. durch Lösen einer Verbindung der allgemeinen Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe von Verbindungen der allgemeinen Formel (I) können in einfacher Weise nach üblichen Verfahren erhalten werden, so z. B. durch Lösen des Metallsalzes in Alkohol, z. B. Ethanol, und Hinzufügen zu Verbindungen der Formel (I). Man kann Metallsalz-Komplexe in bekannter Weise, z. B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubense;

Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Venturia-Arten, wie Venturia inaequalis, Erysiphe-Arten, wie Erysiphe graminis, Cochliobolus-Arten, wie Cochliobolus sativus, Sphaerotheca-Arten, wie Sphaerotheca fuliginea, Pyricularia-Arten, wie Pyricularia oryzae, sowie Pellicularia-Arten, wie Pellicularia sasakii, verwenden.

Ferner eignen sich die erfindungsgemäßen Wirkstoffe sehr gut zur Bekämpfung von Rost sowie Septoria-Arten, wie Septoria nodorum, und Pyrenophora-Arten, wie Pyrenophora teres, an Getreide.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie Mischungen mit Düngemitteln und Wachstumsgulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von0,0001 bis 0,02 %, am Wirkungsort erforderlich.

Herstellung und Verwendung der erfindungsgemäßen Wirkstoffe gehen aus den folgenden Beispielen hervor.

0226916

Herstellungsbeispiele

Beispiel 1

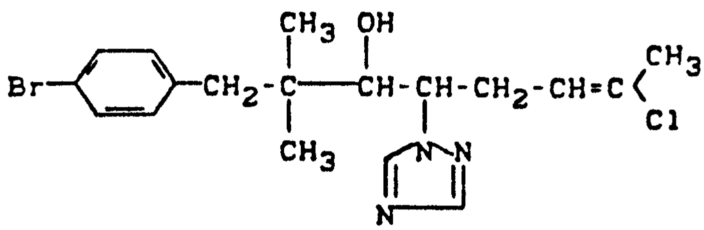

(Verfahren c)

Zu einer Lösung von 9,9 g (0,024 Mol) 1-(4-Bromphenyl)-7-chlor-2,2-dimethyl-4-(1,2,4-triazol-1-yl)-oct-6-en-3-on in 100 ml Methanol wird bei Raumtemperatur unter Rühren eine Lösung von 0,25 g (0,0066 Mol) Natriumborhydrid in 5 ml Wasser zugegeben. Man läßt nach beendeter Zugabe 2 Stunden bei Raumtemperatur nachrühren. Anschließend wird das Reaktionsgemisch mit verdünnter Salzsäure auf pH 6 - 7 gestellt und unter vermindertem Druck eingeengt. Der Rückstand wird in Methylenchlorid aufgenommen. Die organische Phase wird einmal mit Wasser gewaschen, getrocknet und eingeengt.

Man erhält 8,6 g (87 % der Theorie) 1-(4-Bromphenyl)-7-chlor-2,2-dimethyl-4-(1,2,4-triazol-1-yl)-oct-6-en-3-ol vom Schmelzpunkt 93 - 95 °C.

Beispiel 2

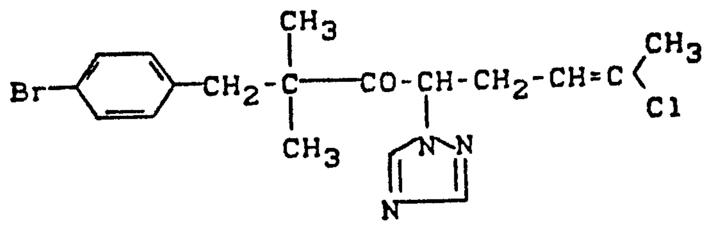

(Verfahren a)

15 g (0,047 Mol) 4-(4-Bromphenyl)-3,3-dimethyl-1-(1,2,4-triazol-1-yl)-butan-2-on, 2,6 g (0,047 Mol) Kaliumhydroxid und 5,9 g 1,3-Dichlorbut-2-en (0,047 Mol) werden in einer Mischung aus 80 ml Dimethylsulfoxid und 10 ml Wasser 2 Stunden bei 50 °C gerührt. Anschließend wird das Reaktionsgemisch in Wasser gegeben und mit Methylenchlorid extrahiert. Die organische Phase wird mit Waser gewaschen, getrocknet und eingeengt. Der Rückstand wird säulenchromatographisch über Kieselgel mit einer Mischung von Essigester/Cyclohexan = 3:1 als Eluierungsmittel gereinigt.

Man erhält 13,7 g (71 % der Theorie) 1-(4-Bromphenyl)-7-chlor-2,2-dimethyl-4-(1,2,4-triazol-1-yl)-oct-6-en-3-on mit dem Brechungsindex $n_D^{20}$ = 1,5587.

Herstellung der Ausgangsprodukte

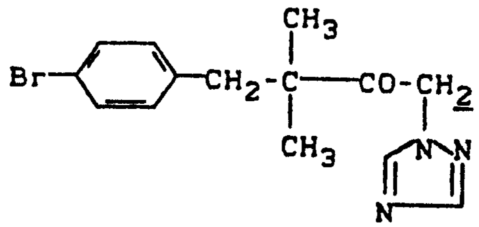

83,5 g (0,25 Mol) 1-Brom-4-(4-bromphenyl)-3,3-dimethyl-butan-2-on werden bei 5 - 10 °C unter Rühren zu einer Mischung von 20,7 g (0,3 Mol) 1,2,4-Triazol und 103,5 g (0,75 Mol) Kaliumcarbonat in 600 ml Aceton zugetropft. Es wird 30 Minuten lang bei 5 - 10 °C und anschließend 8 Stunden lang bei Raumtemperatur nachgerührt. Das Reaktionsgemisch wird filtriert, und das Filtrat wird im Vakuum eingeengt. Der Rückstand wird in Methylenchlorid aufgenommen. Die organische Phase wird mit Wasser gewaschen, getrocknet und eingeengt.

Man erhält 48,1 g (60 % der Theorie) 4-(4-Bromphenyl)-3-dimethyl-1-(1,2,4-triazol-1-yl)-butan-2-on vom Schmelzpunkt 140 °C.

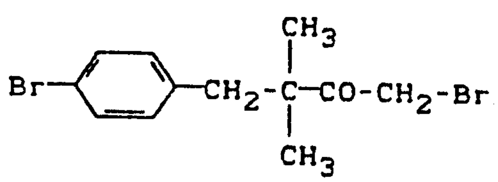

Zu einer Lösung von 65 g (0,25 Mol) 4-(4-Bromphenyl)-3,3-dimethyl-butan-2-on in 200 ml Chloroform werden bei Raumtemperatur unter Rühren 40,8 g (0,25 Mol) Brom zugetropft. Man läßt bei Raumtemperatur 1 Stunde nachrühren und engt anschließend unter vermindertem Druck ein.

Man erhält 83,5 g (100 % der Theorie) 1-Brom-4-(4-bromphenyl)-3,3-dimethyl-butan-2-on mit dem Brechungsindex $n_D^{20}$ = 1,5699.

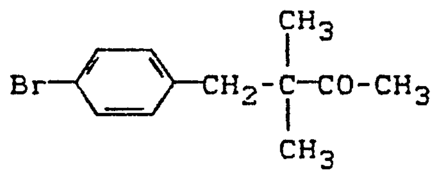

Eine Lösung von 300 g (1,2 Mol) 1-Brom-4-brommethylbenzol und 123,8 g (1,44 Mol) 3-Methylbutan-2-on in 200 ml Toluol läßt man bei Raumtemperatur unter Rühren in ein Gemisch aus 201,6 g (3,6 Mol) Kaliumhydroxid, 12 g Tetrabutylammoniumbromid und 300 ml Toluol einlaufen und 8 Stunden bei gleicher Temperatur nachrühren. Man gibt 500 ml Wasser zu dem Reaktionsgemisch, trennt die Phasen, trocknet die organische Phase und destilliert.

Man erhält 65 g (21 % der Theorie) 4-(4-Bromphenyl)-3,3-dimethyl-butan-2-on vom Siedepunkt 106 - 112 °C / 0,1 mbar.

Beispiel 3

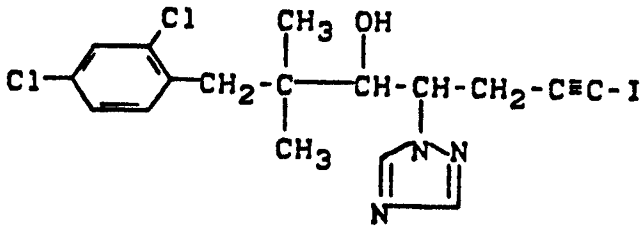

(Verfahren b)

Zu einer Lösung von 5 g (0,014 Mol) 1-(2,4-Dichlorphenyl)-2,2-dimethyl-4-(1,2,4-triazol-1-yl)-hept-6-in-3-ol in 70 ml Methanol werden unter Rühren 7 ml 45-prozentige wäßrige Natronlauge und 4,3 g (0,0168 Mol) Iod gegeben. Es wird 8 Stunden lang bei Raumtemperatur nachgerührt. Das Reaktionsgemisch wird in Wasser gegossen und mit Methylenchlorid extrahiert. Die organische Phase wird abgetrennt, gewaschen, getrocknet und eingeengt. Der Rückstand wird säulenchromatographisch über Kieselgel mit einer Mischung von Essigester/Cyclohexan = 3 : 1 als Eluierungsmittel gereinigt.

Man erhält 4,7 g (70 % der Theorie) 1-(2,4-Dichlorphenyl)-2,2-dimethyl-7-iod-4-(1,2,4-triazol-1-yl)-hept-6-in-3-ol vom Schmelzpunkt 54 - 57 °C.

In analoger Weise und entsprechend den erfindungsgemäßen Verfahren werden die in der folgenden Tabelle 2 aufgeführten Verbindungen der Formel (I)

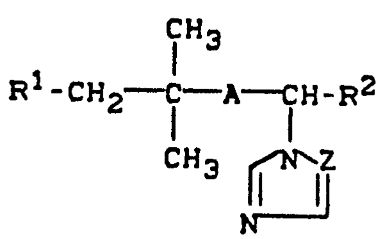

(I)

hergestellt.

Tabelle 2

| Bsp.-Nr. | $R^1$ | A | Z | $R^2$ | Physikal. Daten |
|---|---|---|---|---|---|
| 4 | 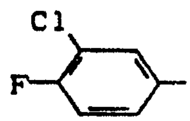 | OH<br>-CH- | N | $-CH_2-C\equiv C-I$ | Fp: 56 °C |
| 5 | 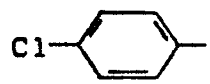 | -CO- | N | $-CH_2-CH=C\langle^{CH_3}_{Cl}$ | $n_D^{20}$= 1,5500 |
| 6 | 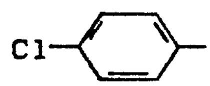 | OH<br>-CH- | N | $-CH_2-CH=C\langle^{CH_3}_{Cl}$ | $n_D^{20}$= 1,5529 |
| 7 | 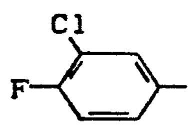 | -CO- | N | $-CH_2-CH=C\langle^{CH_3}_{Cl}$ | $n_D^{20}$= 1,5395 |
| 8 | 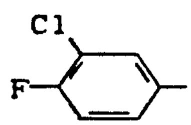 | OH<br>-CH- | N | $-CH_2-CH=C\langle^{CH_3}_{Cl}$ | $n_D^{20}$= 1,5438 |
| 9 | 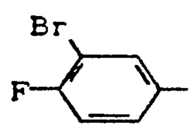 | -CO- | N | $-CH_2-CH=C\langle^{CH_3}_{Cl}$ | $n_D^{20}$= 1,5449 |
| 10 | 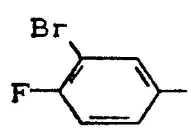 | OH<br>-CH- | N | $-CH_2-CH=C\langle^{CH_3}_{Cl}$ | $n_D^{20}$= 1,5497 |
| 11 | 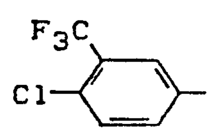 | -CO- | N | $-CH_2-CH=C\langle^{CH_3}_{Cl}$ | $n_D^{20}$= 1,5199 |

Tabelle 2 (Fortsetzung)

| Bsp.-Nr. | $R^1$ | A | Z | $R^2$ | Physikal. Daten |
|---|---|---|---|---|---|
| 12 | 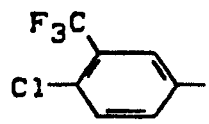 | OH<br>-CH- | N | $-CH_2-CH=C{<}^{CH_3}_{Cl}$ | $n_D^{20}$= 1,5237 |
| 13 | 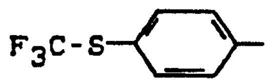 | -CO- | N | $-CH_2-CH=C{<}^{CH_3}_{Cl}$ | $n_D^{20}$= 1,5249 |
| 14 | 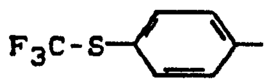 | OH<br>-CH- | N | $-CH_2-CH=C{<}^{CH_3}_{Cl}$ | $n_D^{20}$= 1,5244 |
| 15 | 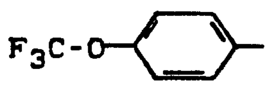 | -CO- | N | $-CH_2-CH=C{<}^{CH_3}_{Cl}$ | $n_D^{20}$= 1,5018 |
| 16 | 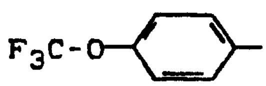 | OH<br>-CH- | N | $-CH_2-CH=C{<}^{CH_3}_{Cl}$ | Fp: 64-66° C |
| 17 | 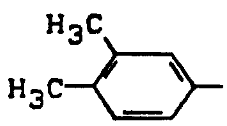 | -CO- | N | $-CH_2-CH=C{<}^{CH_3}_{Cl}$ | Fp: 88 °C |
| 18 | 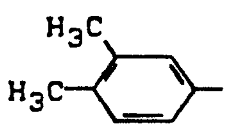 | OH<br>-CH- | N | $-CH_2-CH=C{<}^{CH_3}_{Cl}$ | $n_D^{20}$= 1,5383 |
| 19 | 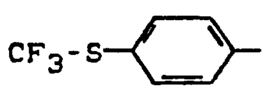 | -CO- | N | $-(CH_2)_6-Br$ | $n_D^{20}$= 1,5215 |
| 20 | 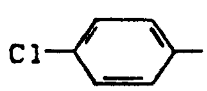 | -CO- | CH | $-CH_2-CH=C{<}^{CH_3}_{Cl}$ | $n_D^{20}$= 1,55 |

Verwendungsbeispiele

In den folgenden Verwendungsbeispielen werden die nachstehend angegebenen Verbindungen als Vergleichssubstanzen eingesetzt:

(A) = 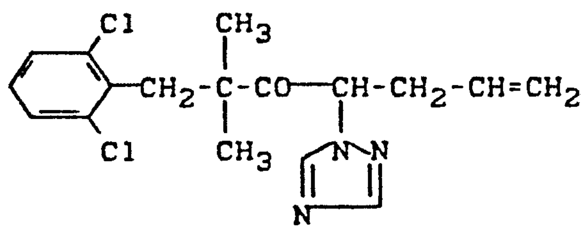

(B) = 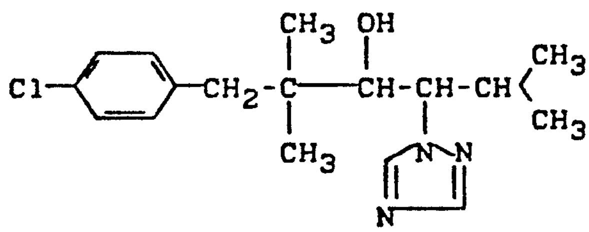

(C) = 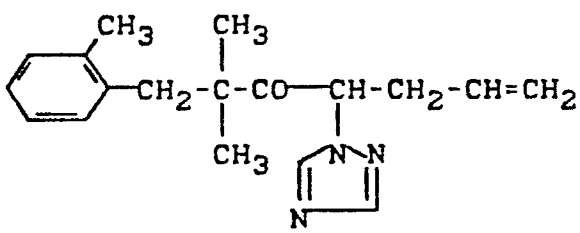

(D) = 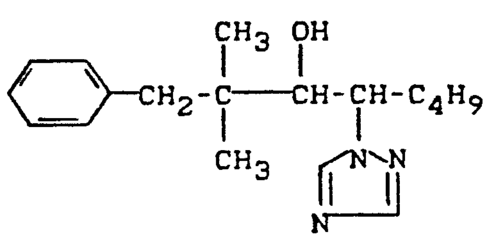

(E) = 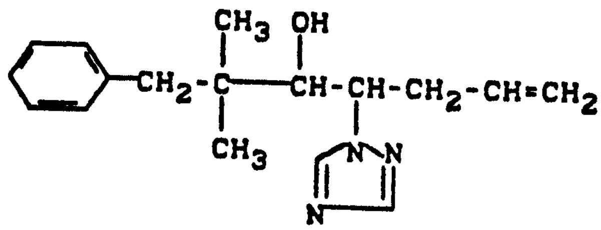

(F) = 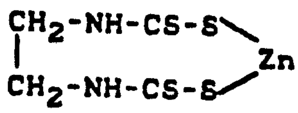

Beispiel A

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigen die in den Beispielen (2), (5), (7), (11) und (13) aufgeführten erfindungsgemäßen Verbindungen eine wesentlich bessere Wirksamkeit als die Vergleichssubstanz (A).

**Beispiel B**

Cochliobolus sativus-Test (Gerste)/protektiv

Lösungsmittel: 100 Gewichtsteile Dimethylformamid
Emulgator : 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20°C und 100 % rel. Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung.

In diesem Test zeigen die in den Beispielen (1), (6), (8), (10), (12), (14), (16) und (18) beschriebenen erfindungsgemäßen Verbindungen eine wesentlich bessere Wirksamkeit als die Vergleichssubstanz (B).

0226916

## Beispiel C

Venturia-Test (Apfel) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolgt die Auswertung.

In diesem Test zeigen die in den Beispielen (2), (13) und (15) beschriebenen erfindungsgemäßen Stoffe eine wesentlich bessere Wirksamkeit als die Vergleichssubstanzen (A) und (C).

Beispiel D

Sphaerotheca-Test (Gurke) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca. 75 % im Gewächshaus aufgestellt.

10 Tage nach der Inokulation erfolgt die Auswertung.

In diesem Test zeigen die in den Beispielen (1), (14), (16) und (18) beschriebenen erfindungsgemäßen Stoffe eine wesentlich bessere Wirksamkeit als die Vergleichssubstanzen (B), (C), (D) und (E).

Beispiel E

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

In diesem Test zeigen die in den Beispielen (1), (6), (8), (12), (14) und (16) beschriebenen erfindungsgemäßen Stoffe eine wesentlich bessere Wirksamkeit als die Vergleichssubstanz (F).

Beispiel F

Pellicularia-Test (Reis)

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und Emulgator und verdünnt das Konzentrat mit Wasser auf die gewünschte Konzentration.

Zur Prüfung auf Wirksamkeit werden junge Reispflanzen im 3- bis 4-Blattstadium tropfnaß gespritzt. Die Pflanzen verbleiben bis zum Abtrocknen im Gewächshaus. Anschließend werden die Pflanzen mit Pellicularia sasakii inokuliert und bei 25°C und 100 % relativer Luftfeuchtigkeit aufgestellt.

5 bis 8 Tage nach der Inokulation erfolgt die Auswertung des Krankheitsbefalles.

In diesem Test zeigen die in den Beispielen (6) und (14) beschriebenen erfindungsgemäßen Stoffe eine wesentlich bessere Wirksamkeit als die Vergleichssubstanz (F).

Patentansprüche

1. 1,4-Disubstituierte 1-Azolyl-3,3-dimethyl-butan-2-on- und -ol-Derivate der Formel

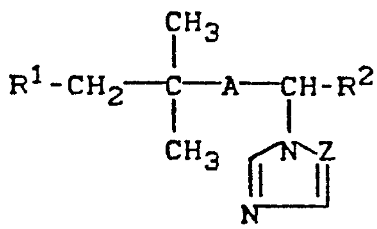

(I)

in welcher

$R^1$ für gegebenenfalls substituiertes Phenyl steht,

A für die Ketogruppe oder eine CH(OH)-Gruppierung steht,

Z für ein Stickstoffatom oder die CH-Gruppe steht und

$R^2$ für Halogenalkyl, Halogenalkenyl oder Halogenalkinyl steht,

sowie deren Säureadditions-Salze und Metallsalz-Komplexe.

2. 1,4-Disubstituierte 1-Azolyl-3,3-dimethyl-butan-2-on- und -ol-Derivate der Formel (I), in denen

$R^1$ für gegebenenfalls einfach bis vierfach, gleich oder verschieden substituiertes Phenyl steht, wobei als Substituenten Halogen, Alkyl, Alkoxy und Alkylthio mit jeweils 1 bis 6 Kohlenstoffatomen, Halogenalkyl, Halogenalkoxy und Halogenalkylthio mit jeweils 1 bis 4 Kohlenstoffatomen und 1 bis 5 gleichen oder verschiedenen Halogenatomen, Nitro, Cyano, Alkoxycarbonyl mit 1 bis 4 Kohlenstoffatomen im Alkoxyteil sowie gegebenenfalls durch Halogen substituiertes Phenyl und Phenoxy genannt seien,

A für die Ketogruppe oder eine CH(OH)-Gruppierung steht,

Z für ein Stickstoffatom oder die CH-Gruppe steht und

$R^2$ für geradkettiges oder verzweigtes Halogenalkyl mit 2 bis 12 Kohlenstoffatomen und Halogenalkenyl mit 3 bis 12 Kohlenstoffatomen mit jeweils 1 bis 3 gleichen oder verschiedenen Halogenatomen oder für geradkettiges oder verzweigtes Halogenalkinyl mit 3 bis 12 Kohlenstoffatomen und einem Halogenatom steht, wobei die Dreifachbindung und das Halogenatom endständig sind.

3. 1,4-Disubstituierte 1-Azolyl-3,3-dimethyl-butan-2-on- und -ol-Derivate der Formel (I), in denen

$R^1$ für gegebenenfalls einfach bis dreifach, gleich oder verschieden substituiertes Phenyl steht, wobei als Substituenten Fluor, Chlor, Brom, Iod, Methyl, Ethyl, Isopropyl, tert.-Butyl, Methoxy, Methylthio, Trifluormethyl, Trifluormethoxy, Chlordifluormethoxy, Trifluormethylthio, Chlordifluormethylthio, Nitro, Cyano, Methoxycarbonyl, gegebenenfalls durch Fluor und/oder Chlor substituiertes Phenyl und gegebenenfalls durch Fluor und/oder Chlor substituiertes Phenoxy genannt seien,

A für die Ketogruppe oder eine CH(OH)-Gruppierung steht,

Z für ein Stickstoffatom oder die CH-Gruppe steht und

$R^2$ für geradkettiges oder verzweigtes Halogenalkyl mit 2 bis 8 Kohlenstoffatomen und 1 bis 3 gleichen oder verschiedenen Halogenatomen wie Fluor, Chlor, Brom und Iod steht, ferner für geradkettiges oder verzweigtes Halogenalkenyl mit 3 bis 8 Kohlenstoffatomen und 1 bis 3 gleichen oder verschiedenen Halogenatomen wie Fluor, Chlor, Brom und Iod steht, oder für geradkettiges oder verzweigtes Halogenalkinyl mit 3 bis 8 Kohlenstoffatomen und 1 Halogenatom, wie Fluor, Chlor, Brom und Iod, steht, wobei die Dreifachbindung und das Halogenatom endständig sind.

4. Verfahren zur Herstellung von 1,4-disubstituierten 1-Azolyl-3,3-dimethyl-butan-2-on- und -ol-Derivaten der Formel

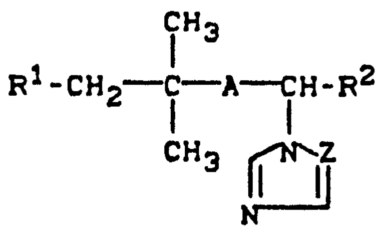

(I)

in welcher

$R^1$ für gegebenenfalls substituiertes Phenyl steht,

A für die Ketogruppe oder eine CH(OH)-Gruppierung steht,

Z für ein Stickstoffatom oder die CH-Gruppe steht und

$R^2$ für Halogenalkyl, Halogenalkenyl oder Halogenalkinyl steht,

sowie von deren Säureadditions-Salzen und Metallsalz-Komplexen, dadurch gekennzeichnet, daß man

(a) Azolyl-ketone der Formel

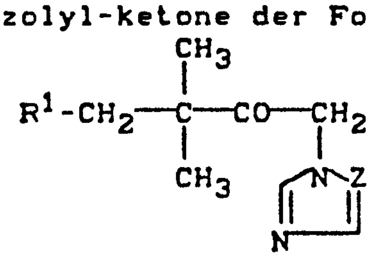

(II)

in welcher

$R^1$ und Z die oben angegebenen Bedeutungen haben,

mit einer Verbindung der Formel

$R^2$—Y (III)

in welcher

$R^2$ die oben angegebene Bedeutung hat und

Y für eine elektronenanziehende Abgangsgruppe steht,

in Gegenwart einer Base und in Gegenwart eines Verdünnungsmittels oder in einem wässrig-organischen Zweiphasensystem in Gegenwart eines Phasentransferkatalysators umsetzt;

oder

(b) Acetylen-Verbindungen der Formel

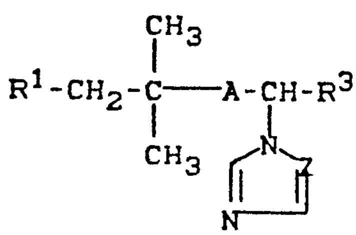

(IV)

in welcher

$R^1$, A und Z die oben angegebenen Bedeutungen haben und

$R^3$ für Alkinyl steht,

mit Halogen in Gegenwart eines Verdünnungsmittels und in Gegenwart einer Base umsetzt, und gegebenenfalls

(c) die nach den Verfahren (a) oder (b) erhaltenen Ketone der Formel

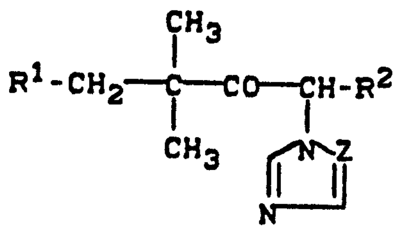

(Ia)

in welcher

$R^1$, Z und $R^2$ die oben angegebenen Bedeutungen haben,

in üblicher Weise reduziert

und gegebenenfalls an die so erhaltenen Verbindungen der Formel (I) anschließend eine Säure oder ein Metallsalz addiert.

5. Fungizide Mittel, gekennzeichnet durch einen Gehalt an mindestens einem 1,4-disubstituierten 1-Azolyl-3,3-dimethyl-butan-2-on- oder -ol-Derivat der Formel (I) bzw. einem Säureadditions-Salz oder Metallsalz-Komplex eines 1,4-disubstituierten 1-Azolyl-3,3-dimethyl-butan-2-on- oder -ol-Derivates der Formel (I).

6. Verwendung von 1,4-disubstituierten 1-Azolyl-3,3-dimethyl-butan-2-on- oder -ol-Derivaten der Formel (I) bzw. von deren Säureadditions-Salzen oder Metallsalz-Komplexen zur Bekämpfung von Pilzen.

7. Verfahren zur Bekämpfung von Pilzen, dadurch gekennzeichnet, daß man 1,4-disubstituierte 1-Azolyl-3,3-dimethyl-butan-2-on- oder -ol-Derivate der Formel (I) bzw. deren Säureadditions-Salze oder Metallsalz-Komplexe auf Pilze und/oder deren Lebensraum ausbringt.

8. Verfahren zur Herstellung von fungiziden Mitteln, dadurch gekennzeichnet, daß man 1,4-disubstituierte 1-Azolyl-3,3-dimethyl-butan-2-on- oder -ol-Derivate der Formel (I) bzw. deren Säureadditions-Salze oder Metallsalz-Komplexe mit Streckmitteln und/oder oberflächenaktiven Stoffen vermischt.

9. 1,4-Disubstituiertes 1-Azolyl-3,3-dimethyl-butan-2-ol-Derivat der Formel

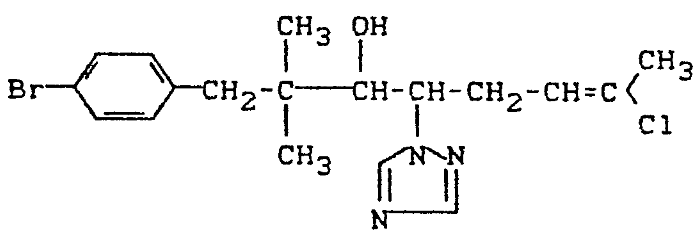

10. 1,4-Disubstituiertes 1-Azolyl-3,3-dimethyl-butan-2-on-Derivat der Formel